# FASCICULE DE BREVET EUROPEEN

(11) **EP 3 269 389 B1**
(45) Date de publication et mention de la délivrance du brevet: **27.05.2020**
(21) Numéro de dépôt: 16178849.2
(22) Date de dépôt: 11.07.2016
(51) Int. Cl.: A61K 45/06, A61K 31/205, A61K 31/221, A61P 15/08

(54) **COMPOSITION COMPRENANT DE LA L-CARNITINE POUR LE TRAITEMENT DE L' INFERTILITE MASCULINE**
ZUSAMMENSETZUNG MIT L-CARNITIN FÜR DIE BEHANDLUNG DER MÄNNLICHEN UNFRUCHTBARKEIT
COMPOSITION COMPRISING L-CARNITINE FOR THE TREATMENT OF MALE INFERTILITY

(43) Date de publication de la demande: 17.01.2018
(73) Titulaire: Laboratoire des Granions, 98000 Monaco (MC)
(72) Inventeur: ACHITE-HENNI, Amine, 92210 Saint-Cloud (FR)
(74) Mandataire: Macquet, Christophe

(56) Documents cités:
- EP-A1- 2 135 621
- WO-A1-99/27925
- WO-A1-03/037343
- FR-A1- 2 964 834
- RASHIDA BM MARIYA: "Comparison of Efficacy of Low Dose and High Dose Antioxidants along with Antibiotics in Idiopathic Oligo, Astheno and Oligoasthenospermia", ANDROLOGY-OPEN ACCESS, vol. 04, no. 01, 25 juin 2015 (2015-06-25) , XP055329405, DOI: 10.4172/2167-0250.1000133
- AFSANEH KHADEMI ET AL.: "The effect of L-carnitine on sperm parameters in patients candidatedfor Intracytoplasmic Sperm Injection", IRANIAN JOURNAL OF REPRODUCTIVE MEDICINE, vol. 2, no. 2, 2004, pages 65-69, XP002765319,
- HESHMATOLLAH SOFIMAJIDPOUR: "Comparison of the Effects of Varicocelectomy and Oral L-carnitine on Sperm Parameters in Infertile Men with Varicocele", JOURNAL OF CLINICAL AND DIAGNOSTIC RESEARCH, avril 2016 (2016-04), XP55329406, ISSN: 2249-782X, DOI: 10.7860/JCDR/2016/18464.7657
- GIORGIO CAVALLINI ET AL: "Reduction in sperm aneuploidy levels in severe oligoasthenoteratospermic patients after medical therapy: a preliminary report", ASIAN JOURNAL OF ANDROLOGY, vol. 14, no. 4, 30 avril 2012 (2012-04-30) , pages 591-598, XP55329881, US ISSN: 1008-682X, DOI: 10.1038/aja.2012.23
- DATABASE WPI Week 200806 Thomson Scientific, London, GB; AN 2008-A87120 XP002765320, "Food additive composition for increasing sperm number and/or improving quality comprises arginine, vitamins B6, B12, C and E, beta carotene, zinc, folic acid, glutathione, coenzyme Q10, L-carnitine, magnesium, selenium, and taurine", & HU 0 600 428 A1 (GAL I) 28 novembre 2007 (2007-11-28)
- VITALI G ET AL: "CARNITINE SUPPLEMENTATION IN HUMAN IDIOPATHIC ASTHENOSPERMIA: CLINICAL RESULTS", DRUGS UNDER EXPERIMENTAL AND CLINICAL RESEARCH, BIOSCIENCE EDIPRINT INC, CH, vol. 11, no. 4, 1 janvier 1995 (1995-01-01), pages 157-159, XP008014041, ISSN: 0378-6501
- Andrea Lenzi ET AL: "Use of carnitine therapy in selected cases of male factor infertility: a double-blind crossover trial", , 1 février 2003 (2003-02-01), XP55329381, DOI: 10.1016/S0015-0282(02) Extrait de l'Internet: URL:http://www.sciencedirect.com/science/a rticle/pii/S0015028202046794/pdfft?md5=c51 f92a84df3ab52cf7a70d4f6ca1abb&pid=1-s2.0-S 0015028202046794-main.pdf [extrait le 2016-12-15]
- BALERCIA G ET AL: "Placebo-controlled double-blind randomized trial on the use of l-carnitine, l-acetylcarnitine, or combined l-carnitine and l-acetylcarnitine in men with idiopathic asthenozoospermia", FERTILITY AND STERILITY, ELSEVIER SCIENCE INC, NEW YORK, NY, USA, vol. 84, no. 3, 1 septembre 2005 (2005-09-01), pages 662-671, XP027699765, ISSN: 0015-0282 [extrait le 2005-09-01]

## Description

### DOMAINE DE L'INVENTION

La présente invention concerne une composition comprenant de la L-carnitine et/ou de l'acétyl-L-carnitine pour le traitement de l'infertilité masculine.

### ART ANTERIEUR

En France, 5 à 10 % des couples rencontrent des difficultés pour avoir un enfant. Dans 21% des cas, l'infertilité masculine apparait à l'origine de ce problème.

Pour observer une fertilité masculine normale, plusieurs conditions doivent être remplies, à savoir:
- une production normale des spermatozoïdes par les testicules (spermatogénèse), en qualité comme en quantité ;
- une bonne circulation des spermatozoïdes au sein des organes génitaux masculins, ce qui implique toute absence d'obstacle au niveau des épididymes, des canaux déférents et de l'urètre ; et
- une éjaculation adéquate.

Tout facteur pouvant entraver l'un de ces mécanismes peut être responsable d'une hypofertilité, voire d'une infertilité (stérilité), chez l'homme.

L'analyse du sperme est l'examen le plus important dans l'évaluation de la fertilité masculine. Le spermogramme mesure avec précision des paramètres comme le nombre de spermatozoïdes, la motilité des spermatozoïdes, la taille et la forme des spermatozoïdes, le volume du sperme, ainsi que le dosage de certaines substances normalement présentes. Les résultats du spermogramme peuvent varier en fonction du stress, de la durée de l'abstinence, de l'absorption d'alcool, de médicaments ou de drogues.

Afin de juger de la qualité du sperme, les données suivantes sont communément (normes Organisation Mondiale de la Santé, intitulées WHO Laboratory Manual for the Examination and Processing of Human Semen, cinquième édition, 2010) prises en considération à partir de l'éjaculat :
- Le volume du sperme est normalement supérieur à 1,5 mL :
   si le volume est supérieur à 6 mL, on parle d'hyperspermie ;
   si le volume est inférieur à 1,5 mL, on parle d'hypospermie.
- La concentration des spermatozoïdes est normalement supérieure ou égale à 15 millions/mL :
   si la concentration est supérieure à 200 millions/mL, on parle de polyspermie ;
   si la concentration est inférieure à 15 millions/mL, on parle d'oligospermie ; on parle d'oligospermie sévère lorsque la concentration est inférieure à 5 millions/mL ; et
   en l'absence totale de spermatozoïdes dans l'éjaculat, on parle d'azoospermie.

Par ailleurs, aux termes de la présente demande, si la concentration est inférieure à 0,5 millions/ML, on parle d'oligospermie très sévère.

Enfin, de manière usuelle, si la concentration est comprise entre 0,01 million/mL et 0,1 million/mL, on parle de cryptozoospermie.
- La mobilité totale des spermatozoïdes est normalement supérieure ou égale à 40% :
   si la mobilité est inférieure à 40%, on parle d'asthénospermie.
- La morphologie normale des spermatozoïdes est supérieure ou égale à 4% :
   si la morphologie est inférieure à 4%, on parle de tératospermie.
- La vitalité des spermatozoïdes est normalement supérieure ou égale à 58%.

Un spermogramme anormal est habituellement associé à une hypofertilité voire une infertilité.

Aussi, l'infertilité masculine est souvent due à un nombre insuffisant de spermatozoïdes, à un défaut de mobilité ou un taux élevé de spermatozoïdes anormaux, ces anomalies pouvant être combinées, par exemple chez des hommes oligoasthénospermiques.

Plus particulièrement, il est connu (article du Dr Jean-Claude Emperaire, Gynécologie endocrinienne du Praticien, 2007) qu'un nombre de spermatozoïdes inférieur à 10 millions/mL d'éjaculat peut notamment être responsable d'une infertilité chez l'homme.

La sévérité de l'infertilité est d'autant plus importante lorsque le nombre de spermatozoïdes par millilitre ne dépasse pas 5 millions (oligospermie sévère) et le pouvoir fécondant devient très faible et tend vers zéro pour les cas de cryptozoospermie. Ce pouvoir fécondant n'est toutefois jamais nul, et tout couple peut connaître des grossesses authentiques attribuables à l'homme malgré une oligospermie très sévère ou une cryptozoospermie.

En pratique, il n'est toutefois pas question d'attendre cette éventualité rare, et il est important de tenter d'améliorer la qualité du sperme pour augmenter les chances d'avoir une fécondation naturelle et ainsi éviter d'avoir recours à des techniques de fécondation *in vitro* très coûteuses et très contraignantes et difficiles pour le patient, qui consistent à ponctionner les testicules ou épididymes de manière à y recueillir les spermatozoïdes qui s'y trouvent, telles que l'ICSI (Intra Cytoplasmic Sperm Injection), ou encore l'IMSI (Intra Morphologic Sperm Injection), ou à un don de sperme, ou encore à l'adoption.

De nombreuses études sur les causes d'infertilité et leurs traitements ont déjà été réalisées.

Il est par exemple connu d'après le document FR2964834 que des antioxydants tels que par exemple la carnitine, le zinc, la vitamine E, le coenzyme Q10 et la vitamine B12 ou encore le glutathion, agissent sur la motilité du sperme. Le zinc et la vitamine B12 sont également décrits pour agir sur la production de sperme.

A cet effet, des études ont été plus particulièrement réalisées mettant en évidence le rôle de la L-carnitine ou ses dérivés, prise seule ou en combinaison avec d'autres composés, sur l'augmentation de la motilité et/ou du nombre des spermatozoïdes chez des hommes hypofertiles.

La publication Balercia et al. (Placebo-controlled double-blind randomized trial on the use of L-carnitine, L-acetylcarnitine, or combined L-carnitine and L-acetylcarnitine in men with idiopathic asthenozoospermia, 2005) met notamment en évidence qu'un traitement de 6 mois avec 3 g/jour de L-carnitine, et plus particulièrement avec 3 g/jour de L-acétylcarnitine ou mieux encore avec 2 g/jour de L-carnitine en combinaison avec 1 g/jour de L-acétylcarnitine, augmente significativement la motilité totale et vers l'avant des spermatozoïdes.

De même, la publication « Lenzi et al., Use of carnitine therapy in selected cases of male factor infertility : a double-blind crossover trial , 2003 » montre l'effet de la L-carnitine (période de traitement de 2 g/jour pendant 2 ^{∗} 2 mois précédée et entrecoupée de périodes de 2 mois sans traitement) sur l'augmentation du nombre et de la motilité totale et vers l'avant des spermatozoïdes chez des patients oligoasthénotératozoospermiques présentant notamment une production de spermatozoïdes comprise entre 10 et 20 millions/mL et une mobilité totale comprise entre 10 et 30% lors de l'inclusion.

D'autres publications tendent à confirmer ces résultats sur le rôle de la L-carnitine prise seule (« Vitali et al., Carnitine supplementation in human idiopathic asthenospermia : Clinical results, 1995 ») ou en combinaison avec de la vitamine E (« Wang et al., L-carnitine : safe and effective for asthenozoospermia, 2010 ») ou encore avec de la L-acétylcarnitine (Lenzi et al., A placebo-controlled double-blind randomized trial of the use of combined L-carnitine and L-acetyl-carnitine treatment in men with asthenozoospermia, 2004); voir également RASHIDA BM MARIYA: "Comparison of Efficacy of Low Dose and High Dose Antioxidants along with Antibiotics in Idiopathic Oligo, Astheno and Oligoasthenospermia", ANDROLOGY-OPEN ACCESS, vol. 04, no. 01, 25 June 2015; AFSANEH KHADEMI ET AL.: "The effect of L-carnitine on sperm parameters in patients candidatedfor Intracytoplasmic Sperm Injection", IRANIAN JOURNAL OF REPRODUCTIVE MEDICINE, vol. 2, no. 2, 2004, pages 65 - 69: ou HESHMATOLLAH SOFIMAJIDPOUR: "Comparison of the Effects of Varicocelectomy and Oral L-carnitine on Sperm Parameters in Infertile Men with Varicocele", JOURNAL OF CLINICAL AND DIAGNOSTIC RESEARCH, April 2016.

Le document brevet publié sous le numéro EP 2 135 621 A décrit un traitement de l'infertilité masculine par la combinaison de deux formulations différentes, qui peuvent être administrées séparément. L'une des formulations contient notamment de l'arginine, de la vitamine A, de la vitamine E, de la vitamine B6 de la vitamine B9, de la vitamine B12, du coenzyme Q10. L'autre formulation contient notamment de la carnitine, de l'arginine, de la vitamine C et du zinc. Les études expérimentales contenues dans ce document sont effectuées sur des couples infertiles. Elles montrent une augmentation de la concentration du sperme pour les patients visés dans l'étude, passant de 26525 ± 25251 mm³ avant traitement à 34200 ± 28254 après traitement, soit une augmentation moyenne de l'ordre de 29%. Ce document ne donne néanmoins pas d'indications sur l'efficacité, ou l'absence d'efficacité, de ces compositions à différentes concentrations spermatiques.

De manière générale, les publications précitées ne mettent pas en évidence ni ne suggèrent un quelconque effet potentialisé de la L-carnitine, prise seule ou en combinaison avec d'autres principes actifs, sur l'augmentation de la production des spermatozoïdes chez des hommes présentant une concentration spermatique inférieure à cinq millions/mL, qui constitue un groupe de patients spécifique, nouveau et différent par rapport aux groupes de patients traités dans l'art antérieur cité.

### RESUME DE L'INVENTION

Considérant ce qui précède, un problème que se propose de résoudre la présente invention est de mettre en œuvre une composition apte à améliorer la production des spermatozoïdes par les testicules chez des hommes produisant peu de spermatozoïdes, plus particulièrement chez des hommes présentant une concentration spermatique inférieure à cinq millions/mL, c'est-à-dire des hommes oligospermiques sévères, cryptozoospermiques ou azoospermiques. La présente invention se propose également de mettre en œuvre une composition apte à améliorer la mobilité des spermatozoïdes. Il est important chez ces hommes hypofertiles voire infertiles, les spermatozoïdes produits pouvant néanmoins atteindre l'éjaculat, de leur permettre de retrouver une fertilité naturelle en augmentant sensiblement le nombre de spermatozoïdes fertiles produits, et ainsi de leur éviter de devoir se tourner vers la fécondation *in vitro,* le don de sperme ou l'adoption pour avoir un enfant.

Ainsi, le Demandeur a découvert, de manière inattendue, que la L-carnitine, et/ou de l'acétyl-L-carnitine en association avec une pluralité d'autres principes actifs agissant en synergie, présente une remarquable activité potentialisée sur la production des spermatozoïdes lorsque les spermatozoïdes ne sont que peu produits chez des hommes présentant une concentration spermatique inférieure à cinq millions/mL, hypofertiles voire infertiles. L'effet activateur de la L-carnitine, prise en association avec une pluralité d'autres principes actifs agissant sur la production de spermatozoïdes, est potentialisé de manière croissante respectivement chez des hommes infertiles présentant moins de trois millions de spermatozoïdes par millilitre, chez des hommes infertiles présentant moins de deux millions de spermatozoïdes par millilitre et chez des hommes infertiles présentant moins d'un million de spermatozoïdes par millilitre.

La solution proposée de l'invention à ce problème technique a pour objet une composition comprenant de la L-carnitine et/ou de l'acétyl-L-carnitine et de l'acide docohexaénoïque et/ou de l'acide eicosapentaénoïque pour utilisation dans le traitement de l'infertilité chez des hommes présentant une concentration spermatique inférieure ou égale à 5 millions de spermatozoïdes par millilitre.

De manière avantageuse, - la composition comprend de l'acide docosahexaénoïque et/ou l'acide eicosapentaénoïque ; - du Coenzyme Q10, de la vitamine E et de la vitamine B6 ; - la concentration spermatique est inférieure ou égale à 3 millions de spermatozoïdes par millilitre, de préférence inférieure ou égale à 2 millions de spermatozoïdes par millilitre, de préférence inférieure ou égale à 1 million de spermatozoïdes par millilitre, de préférence inférieure ou égale à 0,5 million de spermatozoïdes par millilitre ; - la concentration spermatique est inférieure ou égale à 0,1 million de spermatozoïdes par millilitre ; - la concentration spermatique est supérieure à 0,1 million de spermatozoïdes par millilitre ; - la composition est utilisée dans le traitement de l'infertilité chez des hommes présentant une asthénospermie ou une oligoasthénospermie ; - la composition comprend en outre au moins une vitamine et/ou un micronutriment et au moins un oligo-élément ; - la vitamine et/ou le micronutriment est choisi parmi la vitamine E, la vitamine C, la vitamine A, la vitamine B6, la vitamine B8, la vitamine B9, la vitamine B12, la vitamine D, le lycopène, l'arginine, la N-acétyl-cystéine, le coenzyme Q10 et leurs mélanges, de préférence de la vitamine E, de la vitamine B6 et du coenzyme Q10 ; - l'oligo-élément est choisi parmi le zinc, le sélénium, le cuivre, le magnésium, le manganèse, le potassium, l'iode et leurs mélanges, de préférence du zinc et du sélénium ; - la composition comprend du coenzyme Q10, de la vitamine E, de la vitamine B6, du sélénium, du zinc et de l'acide docosahexaénoïque ; - la composition comprend une première formulation contenant du coenzyme Q10, de la vitamine E, de la vitamine B6 et l' acide omega-3, et une seconde formulation contenant du sélénium, du zinc et de la L-carnitine et éventuellement un glucide ; - la première formulation est dosée pour une administration journalière correspondant à 15,0 à 200,0 mg/jour de coenzyme Q10, 6,0 à 24,0 mg/jour de vitamine E, 0,7 à 2,8 mg/jour de vitamine B6 et 600,0 à 1000,0 mg/jour d'acide docosahexaénoïque ; - la seconde formulation est dosée pour une administration journalière correspondant à 10,0 à 100,0 µg/jour de sélénium, 5,0 à 20,0 mg/jour de zinc, 0,5 à 2,0 g/jour de saccharose et 1,0 à 5,0 g/jour de L-carnitine ; - la première formulation se présente sous la forme d'une capsule et/ou la seconde formulation se présente sous la forme d'un sachet à diluer dans un volume de liquide ; - la composition est administrée par voie orale ; - les première et seconde formulations sont administrées avec un intervalle de temps compris entre 8h et 16h, encore plus préférentiellement d'environ 12h, et plus avantageusement la seconde formulation est administrée le matin et la première formulation est administrée le soir ; - la composition est avantageusement administrée pendant une période d'au moins 3 mois; la composition comporte du fructose, de l'acide citrique, du sélénium, du Coenzyme Q, de la vitamine C, du zinc, de l'acide folique et de la vitamine B12 ; - la composition comporte de l'extrait de graines de figues de barbarie, de l'huile de poisson, de la N-acétylcystéine, de l'extrait de fruit de nopal dont de la bétalaine et de la quercétine, de la L-tyrosine, de la carnitine tartrate, de l'extrait de fleur de souci dont de la lutéine, de l'extrait d'Haematococcus pluvialis dont de l'astaxanthine, de l'extrait de fruits d'oranger doux dont de l'hespéridine, de l'extrait de feuilles de thé vert dont des polyphénols, de l'extrait de Dunaliella saline dont des caroténoides, de l'extrait de feuilles d'olivier dont de l'hydroxytyrosol, de l'extrait de fruit d'acérola, de l'extrait d'écorce de pin maritime dont des oligo-proanthocyanidines, du zinc, de vitamine la B9 et de la vitamine B12 ; et - la composition comporte du zinc, de la taurine, de l'arginine, de la vitamine B9, de la vitamine C, de la vitamine E, du sélénium, du Coenzyme Q10, de l'huile de poisson dont des oméga 3.

L'invention et les avantages qui en découlent seront mieux compris à la lecture de la description et des modes de réalisation non limitatifs qui suivent.

### DESCRIPTION DETAILLEE DE L'INVENTION

La composition, de préférence orale, selon l'invention comprend de la L-carnitine et/ou de l'acétyl-L-carnitine en association avec de l'acide docohexaénoïque et/ou de l'acide eicosapentaénoïque, pour le traitement de l'infertilité chez des hommes présentant une oligospermie sévère, une cryptozoospermie ou encore une azoospermie.

La composition selon l'invention est plus particulièrement destinée à des hommes hypofertiles voire infertiles produisant très peu de spermatozoïdes, c'est-à-dire des hommes produisant moins de cinq millions de spermatozoïdes par millilitre, des hommes produisant moins de trois millions de spermatozoïdes par millilitre, des hommes produisant moins de deux millions de spermatozoïdes, chez des hommes produisant moins d'un million de spermatozoïdes par millilitre ou encore chez des hommes dits oligospermiques très sévères produisant moins de 0,5 million de spermatozoïdes par millilitre. La composition selon l'invention est également destinée à des hommes hypofertiles asthénospermiques dont les spermatozoïdes présentent une mobilité inférieure à 40%.

L'oligospermie, l'oligospermie sévère et très sévère, la cryptozoospermie, l'azoospermie et/ou l'asthénospermie selon l'invention sont déterminées après un examen approfondi de l'éjaculat d'un patient donné. Le diagnostic est formalisé à partir des résultats obtenus dans 2 spermogrammes successifs réalisés à 3 mois d'intervalle.

La composition de préférence orale selon l'invention comprend de la L-carnitine et/ou de l'acétyl-L-carnitine et de l'acide docohexaénoïque et/ou de l'acide eicosapentaénoïque.

La L-carnitine est synthétisée à partir de deux acides aminés : la L-lysine et la S-adénosyl-méthionine en présence de vitamine C, magnésium, fer, vitamines B3 et B6. Elle exerce notamment une triple activité : (i) elle permet une production énergétique à partir des acides gras libres via la béta oxydation, les acides gras se liant au coenzyme A pour former les acétyl-CoA et pouvant ainsi traverser la membrane interne de la mitochondrie grâce à un mécanisme enzymatique spécifique utilisant la L-carnitine comme une navette ; (ii) en éliminant les acétyl-CoA, elle exerce un rôle d'antioxydant protégeant l'ADN et la membrane cellulaire contre les radicaux libres ; et (iii) elle favorise la stabilité de la membrane mitochondriale en permettant l'acétylation des phospholipides membranaires. Elle permet ainsi, au niveau des spermatozoïdes, de produire de l'énergie pour leur mobilité, de lutter contre le stress oxydatif et de limiter la dégradation des membranes mitochondriales.

De manière inattendue, l'administration de L-carnitine, augmente de façon particulièrement importante le nombre de spermatozoïdes chez les hommes présentant une concentration spermatique inférieure à cinq millions/mL. De même, l'administration de L-carnitine permet de favoriser la mobilité des spermatozoïdes.

Cette administration est effectuée par voie entérale ou parentérale. Il s'agit préférentiellement d'une administration par voie orale.

Préférentiellement, la composition selon l'invention comprend en outre au moins une vitamine et/ou un micronutriment et au moins un oligo-élément.

A titre d'exemple non limitatif, la vitamine et/ou micronutrimentselon l'invention est choisie parmi la vitamine E, la vitamine C, la vitamine A, la vitamine B6, la vitamine B8, la vitamine B9, la vitamine B12, la vitamine D, le lycopène, l'arginine, la N-acétyl-cystéine et le coenzyme Q10, prise seule ou en combinaison.

A titre d'exemple non limitatif, l'oligo-élément selon l'invention est choisi parmi le zinc, le sélénium, le cuivre, le magnésium, le manganèse, le potassium et l'iode, pris seul ou en combinaison.

La composition selon l'invention peut avantageusement comprendre également des extraits de plantes préférentiellement choisies parmi le pin maritime, en particulier le pycnogénol extrait de l'écorce du pin maritime, le marronnier d'Inde, les polyphénols, la maca ou encore le cornouiller, pris seul ou en combinaison.

De tels extraits végétaux contribuent à améliorer la morphologie, la concentration spermatique, le nombre total et la mobilité des spermatozoïdes, ainsi que plus particulièrement la concentration et le diamètre des veines chez des hommes infertiles avec varicocèle pour le marronnier d'Inde.

Préférentiellement, la composition selon l'invention comprend de la L-carnitine, du coenzyme Q10, de la vitamine E, de la vitamine B6, du sélénium, du zinc, de l'acide docosahexaénoïque ou de l'acide eicosapentaénoïque et un glucide, par exemple le saccharose, comme produit de combinaison. Cette pluralité de principes actifs sont spécifiquement choisis et associés dans la composition selon l'invention pour générer et optimiser la production et la mobilité des spermatozoïdes chez des hommes oligoasthénospermiques, de manière générale la qualité et plus particulièrement la viabilité des spermatozoïdes ainsi produits.

En effet, la synthèse du noyau quinone du coenzyme Q10 à partir de la tyrosine est dépendante de la vitamine B6. La présence de la vitamine B6 permet de maximiser la synthèse endogène du coenzyme Q10. Grâce à cette production endogène, la dose de coenzyme Q10 exogène administrée peut être réduite. En outre, la vitamine E agit en tant que puissant antioxydant diminuant l'impact des radicaux libres au niveau de la membrane plasmique et le plasma séminal, tandis que le coenzyme Q10 agit à deux niveaux, comme antioxydant prévenant la peroxydation de la membrane plasmique du spermatozoïde de sorte à limiter leur dégradation dans le fluide séminal et comme pourvoyeur d'énergie en participant à la synthèse de l'ATP via le cycle de Krebs. La supplémentation en coenzyme Q10, potentialise l'action antioxydante en augmentant la concentration plasmatique des tocophérols (vitamine E). Ce mécanisme passe par le pouvoir régénérateur du coenzyme Q10 sur la vitamine E. En effet, le coenzyme Q10 régénère la vitamine E à partir du radical tocophéryl, après que celle-ci ait rempli ses fonctions antioxydantes. L'effet premier de ces trois actifs qui agissent en corrélation pour limiter le stress oxydatif est supplémenté par l'apport d'acides gras oméga-3, et plus particulièrement l'acide docosahexaénoïque (DHA) et l'acide eicosapentaénoïque, qui font partie des membranes cellulaires et sont responsables du maintien des propriétés de la bicouche lipidique. Ces lipides membranaires du spermatozoïde sont importants pour la fluidité, la flexibilité et la mobilité et donc pour favoriser une fécondation efficace.

Le sélénium et le zinc complètent l'action antioxydante du coenzyme Q10, de la vitamine E et de la vitamine B6 en intervenant plus particulièrement dans la spermatogenèse et la maturation des spermatozoïdes ainsi produits.

Le sélénium est important pour la spermatogenèse puisqu'il est nécessaire à la biosynthèse de la testostérone ainsi qu'à la formation et au développement normal des spermatozoïdes. Le rôle du sélénium dans la spermatogenèse est assuré essentiellement par deux sélénoprotéines : l'hydroperoxide phospholipide glutathion peroxidase (PHGPx/GPx4) et la sélénoprotéine P. La PHGPx/GPx4 possède des fonctions multiples et représente un pivot entre le sélénium, la qualité du sperme et la fertilité masculine. La sélénoprotéine P est une protéine plasmatique nécessaire pour l'apport de sélénium dans les testicules. L'apport en sélénium permet d'augmenter le nombre de spermatozoïdes produits en luttant contre les lésions oxydatives lors de leur maturation et d'améliorer la qualité spermatique en réduisant les possibilités d'anomalies structurales du flagelle.

Le zinc est présent dans les mitochondries et dans le flagelle des spermatozoïdes. L'apport en zinc permet d'éviter des carences affectant la stéroïdogenèse (biosynthèse de la testostérone). L'induction d'une faible carence en zinc provoque des altérations spermatiques expliquées par une réduction de la fonction leydigienne se traduisant par une diminution de la production de spermatozoïdes.

En outre, le saccharose est plus particulièrement utilisé dans la composition selon l'invention pour son rôle pour améliorer le goût et donc l'observance de la prise de la composition selon l'invention et en ce que son hydrolyse en fructose constitue un substrat énergétique pour les spermatozoïdes produits.

De manière préférée, dans le cadre de la présente invention, un produit de combinaison comprenant de la L-carnitine, du coenzyme Q10, de la vitamine E, de la vitamine B6, de l'acide docosahexaénoïque, du sélénium, du zinc et d'un glucide, par exemple le saccharose, signifie que lesdits principes actifs associés peuvent être soit présents au sein d'une même composition, soit présents séparément l'un de l'autre au sein de compositions distinctes. En d'autres termes, ces principes actifs sont destinés à être administrés dans le cadre d'un même traitement, c'est-à-dire sur une période commune de traitement, soit en même temps, en étant compris ou pas au sein d'une seule et même composition, soit en des instants différents. De plus, ils sont de préférence administrés par un même mode d'administration orale dans des compositions identiques ou différentes.

Ainsi, la composition selon l'invention comprend avantageusement une ou plusieurs formulations, comme produit de combinaison pour une utilisation simultanée, séparée ou étalée dans le temps.

Une utilisation simultanée selon l'invention est une administration des composés du produit de combinaison selon l'invention compris dans une seule et même composition ou la prise simultanée de deux formulations distinctes, par exemple une capsule comprenant une première partie de la composition et un sachet dilué dans l'eau pour la seconde partie de la composition.

Une utilisation séparée selon l'invention est une administration, en même temps, des composés du produit de combinaison selon l'invention compris dans au moins deux compositions distinctes, dans une formulation identique ou différente.

Une utilisation étalée dans le temps selon l'invention est une administration successive, des composés du produit de combinaison selon l'invention dans au moins deux compositions distinctes, dans une formulation identique ou différente.

La composition selon l'invention est avantageusement administrable par voie orale, en une ou plusieurs formulations identiques ou différentes. Elle se présente sous une forme galénique quelconque normalement utilisée pour une administration orale et notamment sous la forme de gélule, comprimé, capsule molle, dragée, sachet, tube, flacon, chewing-gum, bille, granule, émulsion, suspension, solution, ampoule, boisson, sirop, poudre, préférentiellement de capsule molle et/ou de sachet.

Avantageusement, considérant les corrélations bénéfiques entre les principes actifs selon l'invention, la composition selon l'invention comprend préférentiellement une première formulation contenant du coenzyme Q10, de la vitamine E, de la vitamine B6 et de l'acide docosahexaénoïque, et une seconde formulation contenant du sélénium, du zinc, du saccharose et de la L-carnitine pour une utilisation séparée ou étalée dans le temps.

La première formulation est préférentiellement dosée pour une administration journalière correspondant à 15,0 à 200,0 mg/jour de coenzyme Q10, 6,0 à 24,0 mg/jour de vitamine E, 0,7 à 2,8 mg/jour de vitamine B6 et 600,0 à 1000,0 mg/jour d'acide docosahexaénoïque, encore plus préférentiellement pour une administration journalière correspondant à 30,0 mg/jour de coenzyme Q10, 12,0 mg/jour de vitamine E, 1,4 mg/jour de vitamine B6 et 800,0 mg/jour d'acide docosahexaénoïque. Ces rapports préférentiels ont été identifiés comme étant adéquats pour résoudre le problème selon l'invention.

Avantageusement, la première formulation est administrée en une prise unique journalière, préférentiellement avant un repas, encore plus préférentiellement le soir avant le repas.

La prise de cette première formulation comprenant notamment l'acide docosahexaénoïque préférentiellement avant un repas limite la stagnation des composés dans l'estomac et permet d'éviter des remontées gastriques désagréables.

Dans un mode de réalisation préféré de la composition selon l'invention, la première formulation se présente sous la forme d'une capsule molle notamment pour administration par voie orale comprenant typiquement de la gélatine, un colorant, et en outre du glycérol. La gélatine est par exemple de la gélatine de poisson. Le colorant est par exemple un oxyde de fer.

A titre d'exemple non limitatif, la capsule molle comprend avantageusement entre 70 et 290 mg de gélatine de poisson pour 30 à 135 mg de glycérol et 2,0 à 10,0 mg d'oxyde de fer.

La seconde formulation est préférentiellement dosée pour une administration journalière correspondant à 10,0 à 100,0 µg/jour de sélénium, 5,0 à 20,0 mg/jour de zinc, et 1,0 à 5,0 g/jour de L-carnitine et éventuellement 0,5 à 2,0 g/jour de saccharose, encore plus préférentiellement pour une administration journalière correspondant à 50,0 µg/jour de sélénium, 10,0 mg/jour de zinc, 1,1 g/jour de saccharose et 3,0 g/jour de L-carnitine. Ces rapports préférentiels ont été identifiés comme étant adéquats pour l'invention.

Avantageusement, la seconde formulation est administrée par voie orale en une prise unique journalière, préférentiellement le matin, encore plus préférentiellement le matin avant le repas.

Dans un mode de réalisation préféré de la composition selon l'invention, la seconde formulation se présente sous la forme d'un sachet à diluer dans un volume de liquide, notamment de l'eau.

A titre d'exemple de procédé de préparation d'une composition selon l'invention, on prépare les 2 formulations constituant une composition préférée selon l'invention suivant les étapes suivantes selon lesquelles :
Concernant la fabrication de la première formulation sous forme de capsule :
- on prépare d'une part le contenu de la capsule par mélange des matières premières sous forme de poudres comprenant du coenzyme Q10, de la vitamine E et de la vitamine B6 à de l'huile de poisson contenant de l'acide docosahexaénoïque dans un réservoir, et de l'autre l'enveloppe de la capsule comprenant de la gélatine de poisson, de l'oxyde de fer et du glycérol à chaud selon un procédé classique de formation de capsules molles ;
- on réalise l'encapsulation du contenu dans l'enveloppe à une température de 20°C avec une humidité relative de 25% ;
- on sèche à température ambiante pour obtenir la première formulation sous forme de capsule ; et
- chaque capsule est dosée pour contenir 10,0 mg de coenzyme Q10, 4,0 mg de vitamine E, 0,47 mg de vitamine B6 et 266,67 mg d'acide docosahexaénoïque.

Concernant la fabrication de la seconde formulation sous forme de sachet à diluer :
- les différents ingrédients sont pesés séparément et le sélénite de sodium est d'abord dilué dans une partie du saccharose ;
- la totalité des ingrédients (incluant la L-carnitine et le zinc) est ensuite introduite dans la cuve de mélange, laquelle va effectuer un premier mélange, par exemple 20 rotations à 8 tours/minute ;
- le mélange est ensuite tamisé et calibré à 1 mm pour passer dans une deuxième cuve qui va effectuer un nouveau mélange, par exemple 10 rotations à 8 tours/minute ;
- la cuve est ensuite reliée à l'ensacheuse et le mélange transféré dans la trémie d'alimentation de l'ensacheuse pour la mise en sachet, chaque sachet contenant 50,0 µg de sélénium, 10,0 mg de zinc, 1,1 g de saccharose et 3,0 g de L-carnitine.

Dans un mode de réalisation préféré de la composition selon l'invention, le régime d'administration entre la première et la seconde formulation comprend un intervalle de temps compris entre 8h et 16h, encore plus préférentiellement d'environ 12h.

Ainsi, à titre d'exemple de mode de réalisation préféré, notamment dans le cas où la première formulation de la composition selon l'invention est formulée en capsules molles dosées comme ci-dessus et où la seconde formulation est formulée en sachets à diluer dosés comme ci-dessus, la posologie indiquée est de trois capsules par jour, en une fois, par exemple le soir pour la première formulation et d'un sachet par jour, par exemple le matin pour la seconde formulation.

La composition selon l'invention est avantageusement administrée pendant une période d'au moins 3 mois, correspondant à une période d'au moins un cycle de production des spermatozoïdes, préférentiellement d'au moins 6 mois, soit plus de deux cycles de production des spermatozoïdes, ou encore pendant 1, 2 voire 3 ans. Avantageusement, une telle durée de traitement d'au moins 3 mois, et préférentiellement d'au moins 6 mois, va permettre de tenter de générer la production de suffisamment de spermatozoïdes présentant une bonne mobilité et qualité par les testicules chez des hommes produisant peu de spermatozoïdes fertiles, de sorte à leur permettre de retrouver une fertilité naturelle ou au moins augmenter sensiblement les chances de trouver des spermatozoïdes fertiles dans l'éjaculat pour une fécondation *in vitro,* et ainsi d'éviter de devoir se tourner vers le don de sperme ou l'adoption pour avoir un enfant.

La composition selon l'invention est nutraceutique ou pharmaceutique.

Un autre objet de la présente invention concerne un complément alimentaire comprenant la composition selon l'invention.

### Exemple 1 : Compositions comprenant de la carnitine selon l'invention pour lutter contre l'infertilité masculine

Les quantités d'ingrédients des compositions 1, 2, 3 et 4 décrites ci-dessous sont les quantités journalières administrées.

### Composition 1

La composition 1 se présente sous forme de deux formulations: en sachet et en capsules. La posologie journalière est d'un sachet le matin et de trois capsules le soir. Trois capsules comprennent 30,0 mg de coenzyme Q10, 12,0 mg de vitamine E, 1,4 mg de vitamine B6 et 1705 mg d'huile de poisson dont 800,0 mg d'acide docosahexaénoïque. Un sachet comprend 50,0 µg de sélénium, 10,0 mg de zinc, 1,1 g de saccharose et 3,0 g de L-carnitine base. Le traitement peut par exemple avoir une durée de 30 jours.

### Composition 2 (ne faisant pas partie de la présente invention)

La composition 2 se présente uniquement sous forme de sachet. La posologie journalière est de deux sachets par jour. Deux sachets contiennent 3,5 g de carnitine fumarate, 1 g d'acétyle-L-carnitine, 2 g de fructose, 100 mg d'acide citrique, 100 µg de sélénium, 40 mg de Coenzyme Q, 180 mg de vitamine C, 20 mg de zinc, 400 µg d'acide folique et 3 µg de vitamine B12. La durée du traitement peut par exemple être de 15 jours.

### Composition 3

La composition 3 se présente sous forme de deux comprimés : un comprimé non coloré et un comprimé coloré. La posologie journalière est de quatre comprimés par jour dont un coloré. Un comprimé coloré contient 100 mg d'extrait de graines de figues de barbarie et 100 mg d'huile de poisson dont 2,30 mg d'acide docosahexaénoïque et 18,20 mg d'acide eicosapentaénoïque. Un comprimé non coloré contient 250 mg de N-acétylcystéine, 200 mg d'extrait de fruit de nopal (dont 0,05 mg de bétalaine et 0.001 mg de quercétine), 150 mg de L-tyrosine, 134,41 mg de carnitine tartrate, 111,12 mg d'extrait de fleur de souci (dont 5,71 mg de lutéine), 85,71 mg d'extrait d'Haematococcus pluvialis (dont 4,29 mg d'astaxanthine), 83,73 mg d'extrait de fruits d'oranger doux (dont 50,24 mg d'hespéridine), 67,47 mg d'extrait de feuilles de thé vert (dont 20,24 mg de polyphénols), 48 mg d'extrait de Dunaliella saline (dont 1,20 mg de caroténoïdes), 47,19 mg d'extrait de feuilles d'olivier (dont 0,47 mg d'hydroxytyrosol), 40,20 mg d'extrait de fruit d'acérola (dont 30 mg de Coenzyme Q10), 20 mg d'extarit d'écorce de pin maritime (dont 19 mg d'oligo-proanthocyanidines), 15 mg de zinc, 12 mg de vitamine E, 1,40 mg de vitamine B6, 178,49 µg de vitamine B9 et 2,50 µg de vitamine B12. Le traitement peut durer par exemple 7 jours.

### Composition 4

La composition 4 se présente sous forme de sachet et de capsule. La posologie journalière est d'un sachet et d'une capsule, de préférence le soir. Un sachet contient 15 mg de zinc, 100 mg de taurine, 3 g de carnitine et 100 mg d'arginine. Une capsule contient 200 µg de vitamine B9, 90 mg de vitamine C, 15 mg de vitamine E, 27,5 µg de sélénium, 30 mg de Coenzyme Q10, 408 mg d'huile de poisson dont 230 mg d'oméga 3 et 200 mg d'acide docosahexaénoïque. Le traitement peut par exemple s'étendre sur 30 jours.

### Exemple 2: Evaluation de l'efficacité de la composition 1 de l'exemple 1 sur l'infertilité masculine

L'invention est plus particulièrement illustrée par les résultats de l'étude observationnelle, multicentrique, ci-après réalisée dans le cadre (i) du Centre de Reproduction du Pôle d'Obstétrique, Reproduction et Gynécologie (ORG) du CHU de Nice (Hôpital Archet II), (ii) du Pôle de Gynécologie-Obstétrique et de Reproduction, service de Biologie de la Reproduction-CECOS du CHU de Bordeaux (Groupe Hospitalier Pellegrin-Maternité) et (iii) de l'Institut de Médecine de la Reproduction de Marseille.

Cette étude a pour objectif d'évaluer l'impact d'un traitement de 6 mois par la composition selon l'invention telle que formulée dans l'exemple de préparation ci-avant sur les paramètres spermatiques principaux, à savoir la numération et la mobilité spermatique, dans une population d'hommes obtenus auprès de 3 centres répartis en France, consultant pour un problème d'infertilité et présentant plus particulièrement une oligospermie, une cryptozoospermie, une azoospermie une asthénospermie ou une oligoasthénospermie.

L'exemple se focalise sur l'analyse de l'impact d'un tel traitement au niveau de la numération chez les patients présentant moins de trois millions de spermatozoïdes par millilitre, et/ou de la mobilité spermatique chez les patients inclus dans l'étude réalisée lors d'au moins deux spermogrammes réalisés avant la visite de début de traitement. La nécessité de réaliser deux spermogrammes différents pour déterminer le caractère pathologique des valeurs d'un spermogramme tient compte de la variabilité personnelle des résultats et constitue une norme OMS.

Lors de la visite de début de traitement, la composition selon l'invention est remise au patient pour un traitement d'une prise quotidienne pendant 6 mois (1 sachet le matin et 3 capsules le soir). Les prises débutent le jour même.

Au cours de la visite de fin de protocole, un spermogramme post traitement est réalisé. L'observance est également évaluée.

Dans la mesure où le spermogramme présente au moins un résultat non évaluable et/ou l'observance n'a pas été respectée, de tels résultats ne sont pas analysés.

La description et l'analyse de la numération spermatique ont été effectuées sur 125 patients dont 73 patients présentent une oligospermie, 5 patients présentent une cryptozoospermie, 4 patients présentent une azoospermie, 10patients présentent une asthénospermie et 33 patients présentent une oligoasthénospermie.

**Tableau 1 : résultats de la numération spermatique**

| | Visite d'inclusion | Visite finale | p value* |
|---|---|---|---|
| Numération(M/ml) Moy±ET(Med[IQR]) | 7,35 ± 9,17 (5,00[1,50;10,00] | 9,91 ± 13,01 (6,00[1,00;12,00] | 0.0088 |

| | | | |
|---|---|---|---|
| *Test de Student pour données appariées | | | |

A l'inclusion, en moyenne les patients avaient une numération spermatique de 7,35 M/ml ± 9,17 contre 9,91M/ml ± 13,01 à la visite finale, soit une augmentation moyenne de 2,56 M/ml ± 10,76, correspondant à une augmentation moyenne de 35%. Cette différence est statistiquement significative.

L'analyse principale a été reprise dans le but d'évaluer l'évolution du nombre de spermatozoïdes chez des sous-groupes de patients : hommes avec une numération spermatique inférieure ou égale à un million/mL à l'inclusion, hommes avec une numération spermatique inférieure ou égale à deux millions/mL à l'inclusion, hommes avec une numération spermatique inférieure ou égale à trois millions/mL à l'inclusion et hommes avec une numération spermatique supérieure à trois millions/mL à l'inclusion.

La description et l'analyse de la numération spermatique chez des hommes avec une numération spermatique inférieure ou égale à un million/mL à l'inclusion ont été réalisées sur 29 patients.

**Tableau 2 : Résultats de la numération spermatique chez les patients qui avaient une numération inférieure ou égale à un million/mL à l'inclusion**

| | Visite d'inclusion | Visite finale | p value* |
|---|---|---|---|
| Numération (M/ml) Moy±ET (Med [IQR]) | 0,20 ± 0,28 (0,10[0,01;0,20] | 2,34 ± 4,62 (0,10[0,01;2,00] | 0.0171 |

| | | | |
|---|---|---|---|
| *Test de Student pour données appariées | | | |

A l'inclusion, en moyenne les patients avaient une numération spermatique de 0,20 M/ml ± 0,28 contre 2,34M/ml ± 4,62 à la visite finale, soit une augmentation moyenne de 2,14 M/ml ± 4,55, correspondant à une augmentation moyenne de 1070%. Cette différence est statistiquement significative.

La description et l'analyse de la numération spermatique chez des hommes avec une numération spermatique inférieure ou égale à deux millions/mL à l'inclusion ont été réalisées sur 45 patients.

**Tableau 3 : Résultats de la numération spermatique chez les patients qui avaient une numération inférieure ou égale à deux millions/mL à l'inclusion**

| | Visite d'inclusion | Visite finale | p value* |
|---|---|---|---|
| Numération (M/ml) | 0,75 ± 0,80 | 3,22 ± 5,00 | 0.0012 |

| | | | |
|---|---|---|---|
| *Test de Student pour données appariées | | | |

A l'inclusion, en moyenne les patients avaient une numération spermatique de 0,75 M/ml ± 0,80 contre 3,22M/ml ± 5,00 à la visite finale, soit une augmentation moyenne de 2,47 M/ml ± 4,77, correspondant à une augmentation moyenne de 329%. Cette différence est statistiquement significative.

La description et l'analyse de la numération spermatique chez des hommes avec une numération spermatique inférieure ou égale à trois millions/mL à l'inclusion ont été réalisées sur 51 patients.

**Tableau 4 : Résultats de la numération spermatique chez les patients qui avaient une numération inférieure ou égale à trois millions/mL à l'inclusion**

| | Visite d'inclusion | Visite finale | p value* |
|---|---|---|---|
| Numération (M/ml) | 1,00 ± 1,02 | 3,70 ± 5,21 | 0.0002 |

| | | | |
|---|---|---|---|
| *Test de Student pour données appariées | | | |

A l'inclusion, en moyenne les patients avaient une numération spermatique de 1,00 M/ml ± 1,02 contre 3,70M/ml ± 5,21 à la visite finale, soit une augmentation moyenne de 2,70 M/ml ± 4,89, correspondant à une augmentation moyenne de 270%. Cette différence est statistiquement significative.

La description et l'analyse de la numération spermatique chez des hommes avec une numération spermatique supérieure à trois millions/mL à l'inclusion ont été réalisées sur 74 patients.

**Tableau 5 : Résultats de la numération spermatique chez les patients qui avaient une numération supérieure à trois millions/mL à l'inclusion**

| | Visite d'inclusion | Visite finale | p value* |
|---|---|---|---|
| Numération (M/ml) | 11,73 ± 9,72 | 14,19 ± 14,94 | 0.1187 |

| | | | |
|---|---|---|---|
| *Test de Student pour données appariées | | | |

A l'inclusion, en moyenne les patients avaient une numération spermatique de 11,73 M/ml ± 9,72 contre 14,19M/ml ± 14,94 à la visite finale, soit une augmentation moyenne de 2,47 M/ml ± 13,43, correspondant à une augmentation moyenne de 21%. Cette différence n'est pas statistiquement significative.

Les résultats démontrent clairement que le traitement pris quotidiennement pendant six mois permet une augmentation de la production de spermatozoïdes chez des patients infertiles. Plus particulièrement, cette augmentation de la concentration spermatique observée suite à la prise du traitement est plus importante chez les patients qui avaient une numération spermatique inférieure ou égale à trois millions/mL que chez les patients qui avaient une numération spermatique supérieure à trois millions/mL. Plus particulièrement, l'effet sur la production de spermatozoïdes est d'autant plus important chez les hommes avec une concentration spermatique inférieure ou égale à deux millions/mL et encore plus particulièrement chez les hommes avec une concentration spermatique inférieure ou égale à un million/mL. En effet, l'augmentation moyenne de la concentration spermatique est de 1070% chez les hommes avec une concentration spermatique inférieure ou égale à un million/mL à l'inclusion, de 329% chez les hommes avec une concentration spermatique inférieure ou égale à deux millions/mL à l'inclusion et de 270% chez les hommes avec une concentration spermatique inférieure ou égale à trois millions/mL à l'inclusion, contre une augmentation moyenne du nombre de spermatozoïdes de 21% chez les hommes avec une concentration spermatique supérieure à trois millions/mL à l'inclusion.

Ces résultats permettent l'identification d'une sous-population particulièrement réceptive au traitement. Les patients infertiles présentant une concentration spermatique inférieure ou égale à trois millions/mL représentent donc une sous-population de patients répondant beaucoup plus efficacement au traitement. L'effet potentialisé du traitement sur cette sous-population présente un caractère surprenant et inattendu.

Enfin, la description et l'analyse de la mobilité des spermatozoïdes ont été réalisées sur 28 patients présentant une mobilité inférieure ou égale à 20% à l'inclusion.

**Tableau 6 : Résultats de la mobilité spermatique chez les patients qui avaient une mobilité inférieure ou égale à 20% à l'inclusion**

| | Visite d'inclusion | Visite finale | p value* |
|---|---|---|---|
| Mobilité(%) Moy±ET (Med [IQR]) | 9,05±7,22 (9,00[2,50;15,00] | 24,61±20,70 (30,00[1,50;40,00] | 0.0002 |

| | | | |
|---|---|---|---|
| *Test de Student pour données appariées | | | |

A l'inclusion, en moyenne les patients avaient une mobilité spermatique de 9,05% ± 7,22 contre 24,61% ± 20,70 à la visite finale, soit une augmentation moyenne absolue de 15,55% ± 19,30, correspondant à une augmentation moyenne relative de 172%. Cette différence est statistiquement significative.

## Revendications

1. Composition comprenant de la L-carnitine et/ou de l'acétyl-L-carnitine et de l'acide docohexaénoïque et/ou de l'acide eicosapentaénoïque destinée à être utilisée dans le traitement de l'infertilité chez des hommes présentant une concentration spermatique inférieure ou égale à 5 millions de spermatozoïdes par millilitre.

2. Composition destinée à être utilisée selon la revendication 1, **caractérisée en ce qu'**elle comprend du Coenzyme Q10, de la vitamine E et de la vitamine B6.

3. Composition destinée à être utilisée selon l'une des revendications précédentes, la concentration spermatique étant inférieure ou égale à 3 millions de spermatozoïdes par millilitre, de préférence inférieure ou égale à 2 millions de spermatozoïdes par millilitre, de préférence inférieure ou égale à 1 million de spermatozoïdes par millilitre, de préférence inférieure ou égale à 0,5 million de spermatozoïdes par millilitre.

4. Composition destinée à être utilisée selon l'une revendications 1 ou 2, la concentration spermatique étant inférieure ou égale à 0,1 million de spermatozoïdes par millilitre.

5. Composition destinée à être utilisée selon l'une des revendications 1 ou 2, la concentration spermatique étant supérieure à 0,1 million de spermatozoïdes par millilitre.

6. Composition destinée à être utilisée selon l'une des revendications précédentes dans le traitement de l'infertilité chez des hommes présentant une asthénospermie ou une oligoasthénospermie.

7. Composition destinée à être utilisée selon l'une des revendications précédentes, comprenant en outre au moins une vitamine et/ou un micronutriment et au moins un oligo-élément.

8. Composition destinée à être utilisée selon la revendication 7, dans laquelle la vitamine et/ou le micronutriment est choisi parmi la vitamine E, la vitamine C, la vitamine A, la vitamine B6, la vitamine B8, la vitamine B9, la vitamine B12, la vitamine D, le lycopène, l'arginine, la N-acétyl-cystéine, le coenzyme Q10 et leurs mélanges, de préférence de la vitamine E, de la vitamine B6 et du coenzyme Q10.

9. Composition destinée à être utilisée selon l'une des revendications 7 ou 8, **caractérisée en ce que** l'oligo-élément est choisi parmi le zinc, le sélénium, le cuivre, le magnésium, le manganèse, le potassium, l'iode et leurs mélanges, de préférence du zinc et du sélénium.

10. Composition destinée à être utilisée selon l'une des revendications précédentes, **caractérisée en ce qu'**elle comprend du coenzyme Q10, de la vitamine E, de la vitamine B6, du sélénium, du zinc et de l'acide docosahexaénoïque.

11. Composition destinée à être utilisée selon la revendication 10, **caractérisée en ce qu'**elle comprend une première formulation contenant du coenzyme Q10, de la vitamine E, de la vitamine B6 et l' acide omega-3, et une seconde formulation contenant du sélénium, du zinc et de la L-carnitine et éventuellement un glucide.

12. Composition destinée à être utilisée selon la revendication 11, **caractérisée en ce que** la première formulation est dosée pour une administration journalière correspondant à 15,0 à 200,0 mg/jour de coenzyme Q10, 6,0 à 24,0 mg/jour de vitamine E, 0,7 à 2,8 mg/jour de vitamine B6 et 600,0 à 1000,0 mg/jour d'acide docosahexaénoïque.

13. Composition destinée à être utilisée selon la revendication 11, **caractérisée en ce que** la seconde formulation est dosée pour une administration journalière correspondant à 10,0 à 100,0 µg/jour de sélénium, 5,0 à 20,0 mg/jour de zinc, 0,5 à 2,0 g/jour de saccharose et 1,0 à 5,0 g/jour de L-carnitine.

14. Composition destinée à être utilisée selon l'une des revendications 11 à 13, **caractérisée en ce que** la première formulation se présente sous la forme d'une capsule et/ou la seconde formulation se présente sous la forme d'un sachet à diluer dans un volume de liquide.

15. Composition destinée à être utilisée selon l'une des revendications précédentes, **caractérisée en ce qu'**elle est administrée par voie orale.

16. Composition destinée à être utilisée selon l'une des revendications 11 à 14, dans laquelle les première et seconde formulations sont administrées avec un intervalle de temps compris entre 8h et 16h, encore plus préférentiellement d'environ 12h, et plus avantageusement la seconde formulation est administrée le matin et la première formulation est administrée le soir.

17. Composition destinée à être utilisée selon l'une des revendications précédentes, **caractérisée en ce qu'**elle est administrée pendant une période d'au moins 3 mois.

18. Composition destinée à être utilisée selon la revendication 1, **caractérisée en ce qu'**elle comporte du fructose, de l'acide citrique, du sélénium, du Coenzyme Q, de la vitamine C, du zinc, de l'acide folique et de la vitamine B12.

19. Composition destinée à être utilisée selon l'une des revendications 1 ou 2, **caractérisée en ce qu'**elle comporte de l'extrait de graines de figues de barbarie, de l'huile de poisson, de la N-acétylcystéine, de l'extrait de fruit de nopal dont de la bétalaine et de la quercétine, de la L-tyrosine, de la carnitine tartrate, de l'extrait de fleur de souci dont de la lutéine, de l'extrait d'Haematococcus pluvialis dont de l'astaxanthine, de l'extrait de fruits d'oranger doux dont de l'hespéridine, de l'extrait de feuilles de thé vert dont des polyphénols, de l'extrait de Dunaliella saline dont des caroténoïdes, de l'extrait de feuilles d'olivier dont de l'hydroxytyrosol, de l'extrait de fruit d'acérola, de l'extrait d'écorce de pin maritime dont des oligo-proanthocyanidines, du zinc, de la vitamine B9 et de la vitamine B12.

20. Composition destinée à être utilisée selon la revendication 1, **caractérisée en ce qu'**elle comporte du zinc, de la taurine, de l'arginine, de la vitamine B9, de la vitamine C, de la vitamine E, du sélénium, du Coenzyme Q10, de l'huile de poisson dont des oméga 3.

## Patentansprüche

1. Zusammensetzung, L-Carnitin und/ oder Acetyl-L-Carnitin und Docosahexaensäure und/ oder Eicosapentaensäure umfassend, dazu bestimmt, bei der Behandlung der Unfruchtbarkeit bei Männern verwendet zu werden, die eine Spermienkonzentration kleiner oder gleich 5 Millionen Spermien je Milliliter aufweisen.

2. Zusammensetzung, die dazu bestimmt ist, gemäß Anspruch 1 verwendet zu werden, **dadurch gekennzeichnet, dass** sie Coenzym Q10, Vitamin E und Vitamin B6 umfasst.

3. Zusammensetzung, die dazu bestimmt ist, gemäß einem der vorstehenden Ansprüche verwendet zu werden, wobei die Spermienkonzentration kleiner oder gleich 3 Millionen Spermien je Milliliter, vorzugsweise kleiner oder gleich 2 Millionen Spermien je Milliliter, vorzugsweise kleiner oder gleich 1 Million Spermien je Milliliter, vorzugsweise kleiner oder gleich 0,5 Millionen Spermien je Milliliter ist.

4. Zusammensetzung, die dazu bestimmt ist, gemäß einem der Ansprüche 1 oder 2 verwendet zu werden, wobei die Spermienkonzentration kleiner oder gleich 0,1 Millionen Spermien je Milliliter ist.

5. Zusammensetzung, die dazu bestimmt ist, gemäß einem der Ansprüche 1 oder 2 verwendet zu werden, wobei die Spermienkonzentration größer als 0,1 Millionen Spermien je Milliliter ist.

6. Zusammensetzung, die dazu bestimmt ist, gemäß einem der vorstehenden Ansprüche bei der Behandlung der Unfruchtbarkeit bei Männern verwendet zu werden, die eine Asthenozoospermie oder eine Oligoasthenozoospermie aufweisen.

7. Zusammensetzung, die dazu bestimmt ist, gemäß einem der vorstehenden Ansprüche verwendet zu werden, weiter mindestens ein Vitamin und/ oder einen Mikronährstoff und mindestens ein Oligoelement umfassend.

8. Zusammensetzung, die dazu bestimmt ist, gemäß Anspruch 7 verwendet zu werden, wobei das Vitamin und/ oder der Mikronährstoff ausgewählt wird aus Vitamin E, Vitamin C, Vitamin A, Vitamin B6, Vitamin B8, Vitamin B9, Vitamin B12, Vitamin D, Lycopin, Arginin, N-Acetylcystein, Coenzym Q10 und deren Mischungen, vorzugsweise Vitamin E, Vitamin B6 und Coenzym Q10.

9. Zusammensetzung, die dazu bestimmt ist, gemäß Anspruch 7 oder 8 verwendet zu werden, **dadurch gekennzeichnet, dass** das Oligoelement aus Zink, Selen, Kupfer, Magnesium, Mangan, Kalium, Iod, und deren Mischungen, vorzugsweise Zink und Selen, ausgewählt wird.

10. Zusammensetzung, die dazu bestimmt ist, gemäß einem der vorstehenden Ansprüche verwendet zu werden, **dadurch gekennzeichnet, dass** sie Coenzym Q10, Vitamin E, Vitamin B6, Selen, Zink und Docosahexaensäure umfasst.

11. Zusammensetzung, die dazu bestimmt ist, gemäß Anspruch 10 verwendet zu werden, **dadurch gekennzeichnet, dass** sie eine erste Formulierung umfasst, die Coenzym Q10, Vitamin E, Vitamin B6 und Omega-3-Säure enthält, und eine zweite Formulierung, die Selen, Zink und L-Carnitin und eventuell Kohlenhydrat enthält.

12. Zusammensetzung, die dazu bestimmt ist, gemäß Anspruch 11 verwendet zu werden, **dadurch gekennzeichnet, dass** die erste Formulierung für eine tägliche Verabreichung entsprechend 15,0 bis 200,0 mg/Tag Coenzym Q10, 6,0 bis 24,0 mg/ Tag Vitamin E, 0,7 bis 2,8 mg/ Tag Vitamin B6 und 600,0 bis 1000,0 mg/ Tag Docosahexaensäure dosiert ist.

13. Zusammensetzung, die dazu bestimmt ist, gemäß Anspruch 11 verwendet zu werden, **dadurch gekennzeichnet, dass** die erste Formulierung für eine tägliche Verabreichung entsprechend 10,0 bis 100,0 µg/Tag Selen, 5,0 bis 20,0 mg/ Tag Zink, 0,5 bis 2,0 g/ Tag Saccharose und 1,0 bis 5,0 g/ Tag L-Carnitin dosiert ist.

14. Zusammensetzung, die dazu bestimmt ist, gemäß einem der Ansprüche 11 bis 13 verwendet zu werden, **dadurch gekennzeichnet, dass** sich die erste Formulierung in Form einer Kapsel präsentiert und/ oder sich die zweite Formulierung in Form eines Beutels zum Verdünnen in einem Flüssigkeitsvolumen präsentiert.

15. Zusammensetzung, die dazu bestimmt ist, gemäß einem der vorstehenden Ansprüche verwendet zu werden, **dadurch gekennzeichnet, dass** sie oral verabreicht wird.

16. Zusammensetzung, die dazu bestimmt ist, gemäß einem der Ansprüche 11 bis 14 verwendet zu werden, wobei die erste und zweite Formulierung in einem Zeitintervall verabreicht werden, das zwischen 8 Stunden und 16 Stunden, bevorzugter etwa 12 Stunden verabreicht werden und die zweite Formulierung vorteilhafterweise morgens verabreicht wird und die erste Formulierung abends verabreicht wird.

17. Zusammensetzung, die dazu bestimmt ist, gemäß einem der vorstehenden Ansprüche verwendet zu werden, **dadurch gekennzeichnet, dass** sie während eines Zeitraums von mindestens 3 Monaten verabreicht wird.

18. Zusammensetzung, die dazu bestimmt ist, gemäß Anspruch 1 verwendet zu werden, **dadurch gekennzeichnet, dass** sie Fructose, Zitronensäure, Selen, Coenzym Q, Vitamin C, Zink, Folsäure, und Vitamin B12 beinhaltet.

19. Zusammensetzung, die dazu bestimmt ist, gemäß einem der Ansprüche 1 oder 2 verwendet zu werden, **dadurch gekennzeichnet, dass** sie Kaktusfeigen-Kernextrakt, Fischöl, N-Acetylcystein, Nopal-Fruchtextrakt, darunter Betalain und Quercetin, L-Tyrosin, Canitintartrat, Ringelblumen-Extrakt, darunter Lutein, Haematococcus-pluvialis-Extrakt, darunter Astaxanthin, Orangenbaum-Extrakt, darunter Hesperidin, Extrakt aus grünen Teeblättern, darunter Polyphenole, Extrakt aus Dunaliella Salina Algen, darunter Carotenoiden, Extrakt aus dem Olivenbaumblatt, darunter Hydroxytyrosol, Acerola-Fruchtextrakt, Seekieferrindenextrakt, darunter Oligoproanthocyanidinen, Zink, Vitamin B9 und Vitamin B12 beinhaltet.

20. Zusammensetzung, die dazu bestimmt ist, gemäß Anspruch 1 verwendet zu werden, **dadurch gekennzeichnet, dass** sie Zink, Taurin, Arginin, Vitamin B9, Vitamin C, Vitamin E, Selen, Coenzym Q10, Fischöl, darunter Omega 3 beinhaltet.

## Claims

1. A composition comprising L-carnitine and/or acetyl-L-carnitine and docohexaenoic acid and/or eicosapentaenoic acid intended to be used in the treatment of infertility in men having a sperm concentration less than or equal to 5 million spermatozoa per millilitre.

2. The composition intended to be used according to claim 1, **characterised in that** it comprises Coenzyme Q10, vitamin E and vitamin B6.

3. The composition intended to be used according to one of the preceding claims, the sperm concentration being less than or equal to 3 million spermatozoa per millilitre, preferably less than or equal to 2 million spermatozoa per millilitre, preferably less than or equal to 1 million spermatozoa per millilitre, preferably less than or equal to 0.5 million spermatozoa per millilitre.

4. The composition intended to be used according to one of claims 1 or 2, the sperm concentration being less than or equal to 0.1 million spermatozoa per millilitre.

5. The composition intended to be used according to one of claims 1 or 2, the sperm concentration being greater than 0.1 million spermatozoa per millilitre.

6. The composition intended to be used according to one of the preceding claims in the treatment of infertility in men having asthenospermia or oligoasthenospermia.

7. The composition intended to be used according to one of the preceding claims, further comprising at least one vitamin and/or one micronutrient and at least one trace element.

8. The composition intended to be used according to claim 7, wherein the vitamin and/or the micronutrient is selected from vitamin E, vitamin C, vitamin A, vitamin B6, vitamin B8, vitamin B9, vitamin B12, vitamin D, lycopene, arginine, N-acetyl-cysteine, coenzyme Q10 and mixtures thereof, preferably vitamin E, vitamin B6 and coenzyme Q10.

9. The composition intended to be used according to one of claims 7 or 8, **characterised in that** the trace element is selected from zinc, selenium, copper, magnesium, manganese, potassium, iodine and mixtures thereof, preferably zinc and selenium.

10. The composition intended to be used according to one of the preceding claims, **characterised in that** it comprises coenzyme Q10, vitamin E, vitamin B6, selenium, zinc and docosahexaenoic acid.

11. The composition intended to be used according to claim 10, **characterised in that** it comprises a first formulation containing coenzyme Q10, vitamin E, vitamin B6, and omega-3 acid, and a second formulation containing selenium, zinc and L-carnitine and optionally a carbohydrate.

12. The composition intended to be used according to claim 11, **characterised in that** the first formulation is dosed for a daily administration corresponding to 15.0 to 200.0 mg/day of coenzyme Q10, 6.0 to 24.0 mg/day of vitamin E, 0.7 to 2.8 mg/day of vitamin B6 and 600.0 to 1000.0 mg/day of docosahexaenoic acid.

13. The composition intended to be used according to claim 11, **characterised in that** the second formulation is dosed for a daily administration corresponding to 10.0 to 100.0 µg/day of selenium, 5.0 to 20.0 mg/day of zinc, 0.5 to 2.0 g/day of sucrose and 1.0 to 5.0 g/day of L-carnitine.

14. The composition intended to be used according to one of claims 11 to 13, **characterised in that** the first formulation is in the form of a capsule and/or the second formulation is in the form of a sachet to be diluted in a volume of liquid.

15. The composition intended to be used according to one of the preceding claims, **characterised in that** it is administered orally.

16. The composition intended to be used according to one of claims 11 to 14, wherein the first and second formulations are administered with a time interval comprised between 8h and 16h, even more preferably of about 12h, and more advantageously the second formulation is administered in the morning and the first formulation is administered in the evening.

17. The composition intended to be used according to one of the preceding claims, **characterised in that** it is administered for a period of at least 3 months.

18. The composition intended to be used according to claim 1, **characterised in that** it includes fructose, citric acid, selenium, Coenzyme Q, vitamin C, zinc, folic acid and vitamin B12.

19. The composition intended to be used according to one of claims 1 or 2, **characterised in that** it includes prickly pear seed extract, fish oil, N-acetylcysteine, nopal fruit extract including betulin and quercetin, L-tyrosine, carnitine tartrate, marigold flower extract including lutein, Haematococcus pluvialis extract including astaxanthin, sweet orange fruit extract including hesperidin, green tea leaf extract including polyphenols, Dunaliella salina extract including carotenoids, olive leaf extract including hydroxytyrosol, acerola fruit extract, maritime pine bark extract including oligo-proanthocyanidins, zinc, vitamin B9 and vitamin B12.

20. The composition intended to be used according to claim 1, **characterised in that** it includes zinc, taurine, arginine, vitamin B9, vitamin C, vitamin E, selenium, Coenzyme Q10, fish oil including omega 3.
